# EUROPÄISCHE PATENTSCHRIFT

(11) **EP 3 518 899 B1**
(45) Veröffentlichungstag und Bekanntmachung des Hinweises auf die Patenterteilung: **15.07.2020**
(21) Anmeldenummer: 17783760.6
(22) Anmeldetag: 25.09.2017
(51) Int. Cl.: A61K 9/10, A61K 9/70

(54) **VERFAHREN ZUM FIXIEREN PFLANZLICHER WIRKSTOFFE AN EINEM NICHTMETALLISCHEN SUBSTRAT**
METHOD FOR IMMOBILISING PLANT ACTIVE SUBSTANCES AN A NON-METALLIC SUBSTRATE
PROCÉDÉ POUR FIXER DES SUBSTANCES ACTIVES VÉGÉTALES SUR UN SUBSTRAT NON MÉTALLIQUE

(30) Priorität: 27.09.2016 DE 102016118274
(43) Veröffentlichungstag der Anmeldung: 07.08.2019
(73) Patentinhaber: Fraunhofer-Gesellschaft zur Förderung der angewandten Forschung e.V., 80686 München (DE)
(72) Erfinder: DIETZE, Marleen, 01277 Dresden (DE); KEMPER, Benjamin, 01127 Dresden (DE); KUBUSCH, Jörg, 01279 Dresden (DE)
(86) Internationale Anmeldenummer: PCT/EP2017/074269
(87) Internationale Veröffentlichungsnummer: WO 2018/060150

(56) Entgegenhaltungen:
- EP-A1- 0 914 820
- EP-A1- 1 889 608
- DE-A1- 10 121 471

## Beschreibung

Das Funktionalisieren von Oberflächen erlangt eine zunehmende Bedeutung. Durch verschiedenste Techniken und Verfahren können beispielsweise die Oberflächenenergie, Biokompatibilität und Korrosionsbeständigkeit von Oberflächen beeinflusst sowie dem Material neue Eigenschaften, wie zum Beispiel eine antimikrobielle Wirksamkeit, verliehen werden. Letzteres wird durch das Auftragen unterschiedlicher Substanzen, die diese Eigenschaft aufweisen, erreicht. Neben herkömmlichen Stoffen anorganischen Ursprungs, wie zum Beispiel Silber oder Aluminium, bietet die Pflanzenwelt ein großes Repertoire beispielsweise antimikrobieller Wirkstoffe. Vorteilhaft ist der Einsatz derartiger Stoffe insbesondere in der Humanmedizin, da geringe Nebenwirkungen und seltener auftretende Wechselwirkungen mit anderen Arzneimitteln vorkommen sowie die Akzeptanz der Patienten gegenüber Produkten mit pflanzlichen Bestandteilen als sehr hoch einzustufen ist. Zudem sind pflanzliche Wirkstoffe überwiegend atoxisch. Unter dem Begriff pflanzlicher Wirkstoffe sollen nachfolgend pflanzliche Inhaltsstoffe verstanden werden, die eine medizinische, gesundheitsfördernde und/oder kosmetische Wirkung entfalten.

Beim Funktionalisieren einer Oberfläche mit pflanzlichen Wirkstoffen müssen diese an der Oberfläche fixiert werden. Herausforderungen dabei bestehen vor allem darin, eine stabile Bindung der pflanzlichen Wirkstoffe an der Oberfläche zu schaffen sowie den Funktionserhalt der Wirkstoffe sicher zu stellen.

Aus DE 101 21 471 A1 sind Verfahren zum Herstellen von Wundauflagen in Form von Matrixpflastern bekannt, bei welchem pflanzliche Wirkstoffe in flüssiger oder gelöster Form auf die selbstklebende Oberfläche einer Polymermatrix mittels Drucken oder Sprühen aufgetragen werden. Verfahren zum Herstellen wirkstoffhaltiger Pflaster sind auch in DE 197 49 467 A1 offenbart. Hierbei wird ein Wirkstoff in eine Klebemasse eingearbeitet und die auf die mit dem Wirkstoff dotierte Klebemasse auf einem Trägermaterial aufgetragen. Beschränkt sind diese Vorgehensweisen dadurch, dass lediglich Substrate in Verbindung mit einer Klebemasse oder zumindest einer klebenden Oberfläche verwendet werden können. Eingebettet in einer Klebemasse kann der Wirkstoff auch nur begrenzt seine Wirksamkeit an einer Wunde entfalten.

Ein Verfahren zum Herstellen eines Hydrogels ist in EP 1 889 608 A1 vorgeschlagen, bei welchem zunächst ein biokompatibles Polymer, ein Polyalkohol und ein pflanzlicher Extrakt miteinander vermischt werden. Das entstehende Gemisch wird anschließend gefroren und wieder aufgetaut, um eine Vernetzung zu erzielen welche abschließend noch verstärkt wird, indem das Gemisch einer Strahlung ausgesetzt wird. Diese Vorgehensweise ist jedoch nur zum Erzielen gelförmiger Endprodukte anwendbar.

Der Erfindung liegt daher das technische Problem zugrunde, ein Verfahren zum Fixieren pflanzlicher Wirkstoffe an einem nichtmetallischen Substrat zu schaffen, mittels dessen die Nachteile aus dem Stand der Technik überwunden werden können. Insbesondere soll es mit dem erfindungsgemäßen Verfahren auch möglich sein, pflanzliche Wirkstoffe an Polymeren und textilen Geweben zu fixieren.

Die Lösung des technischen Problems ergibt sich durch Gegenstände mit den Merkmalen des Patentanspruchs 1. Weitere vorteilhafte Ausgestaltungen der Erfindung ergeben sich aus den abhängigen Ansprüchen.

Beim erfindungsgemäßen Verfahren zum Fixieren pflanzlicher Wirkstoffe an einem Oberflächenbereich eines nichtmetallischen Substrates wird ein pflanzlicher Extrakt auf dem Oberflächenbereich des Substrates aufgetragen und nachfolgend der Oberflächenbereich des Substrates mit niederenergetischen Elektronen eines Elektronenstrahls beaufschlagt.

Unter einem pflanzlichen Extrakt sollen verstanden werden, Auszüge, Konzentrate, Lösungen und Zubereitungen von flüssiger, halbfester oder fester Beschaffenheit, die durch ein Extraktionsverfahren aus getrockneten und/oder nicht getrockneten Pflanzenteilen gewonnen werden. Dabei umfasst das Herstellen eines pflanzlichen Extraktes auch das Entfernen eines Lösungsmittels, falls ein solches beim Extraktionsverfahren zum Einsatz gelangte. Das Entfernen des Lösungsmittels kann beispielsweise mittels Destillation, Verdampfen, Kristallisation oder dergleichen erfolgen.

Überraschend hat sich gezeigt, dass das Beaufschlagen einer Substratoberfläche mit niederenergetischen Elektronen, nachdem ein pflanzlicher Extrakt auf der Substratoberfläche aufgetragen wurde, nicht nur eine sterilisierende Wirkung auf die bestrahlten Gegenstände ausüben kann, wie es aus dem Stand der Technik bekannt ist, sondern dass dieser Vorgang mit einer verbesserten Haftung von Bestandteilen des pflanzlichen Extraktes und den darin enthaltenen pflanzlichen Wirkstoffen an der Substratoberfläche einhergeht. Es wird vermutet, dass durch das Beaufschlagen einer nichtmetallischen Substratoberfläche mit niederenergetischen Elektronen die Substratoberfläche dahingehend aktiviert wird, dass offene und sehr reaktive Bindungsstellen geschaffen werden, die eine Verbindung mit Bestandteilen des pflanzlichen Extraktes und den darin enthaltenen pflanzlichen Wirkstoffen eingehen, der auf der Substratoberfläche aufgetragen wurde. Das erfindungsgemäße Verfahren ist daher besonders für das Herstellen von Wundauflagen oder medizinischen Gegenständen geeignet, die mit einem menschlichen oder tierischen Körper in Kontakt gelangen. Mit dem erfindungsgemäßen Verfahren lassen sich beispielsweise auch Textilien oder Gegenstände des täglichen Gebrauchs, wie zum Beispiel Taschentücher, mit antibakteriellen Oberflächen ausstatten, indem pflanzliche Wirkstoffe daran fixiert werden.

Da die Bestandteile eines pflanzlichen Extraktes nicht beschädigt und somit in deren Wirksamkeit eingeschränkt werden sollen, gelangen beim erfindungsgemäßen Verfahren lediglich niederenergetische Elektronen zur Anwendung. Beim erfindungsgemäßen Verfahren werden beschleunigte Elektronen mit einer Energie von 100 keV bis 500 keV für das Beaufschlagen eines Oberflächenbereichs des Substrates verwendet und das Beaufschlagen des Oberflächenbereiches des Substrates mit niederenergetischen Elektronen erfolgt mit einer Dosis von 1 kGy bis 200 kGy. Dabei erfolgt das Beaufschlagen der Substratoberfläche mit niederenergetischen Elektronen durch die auf der Substratoberfläche aufgetragene Schicht des pflanzlichen Extraktes hindurch.

Wegen des Einsatzes beschleunigter Elektronen ist das erfindungsgemäße Verfahren für nichtmetallische Substrate geeignet. Als Substratmaterialien sind beispielsweise synthetische Polymere, Biopolymere oder Textilien geeignet.

Pflanzliche Extrakte, welche in flüssiger, gelförmiger oder auch fester Form bekannt sind, können auf einen Oberflächenbereich eines Substrates, beispielsweise durch Drucken, Streichen, Rakeln, Sprühen oder mittels Eintauchen des Substrates in den pflanzlichen Extrakt erfolgen, wobei der pflanzliche Extrakt als Konzentrat oder in gelöster Form verwendet werden kann. Ebenfalls für das erfindungsgemäße Verfahren geeignet sind Gemische aus verschiedenen pflanzlichen Extrakten.

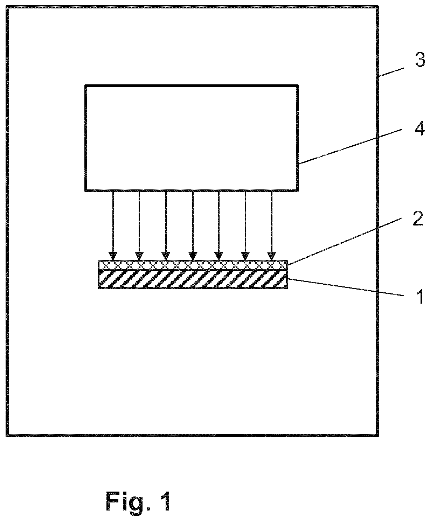

Die vorliegende Erfindung wird nachfolgend anhand eines Ausführungsbeispiels näher erläutert. Fig. 1 zeigt in einer schematischen Darstellung eine Anordnung zum Ausführen des erfindungsgemäßen Verfahrens. Für das Herstellen einer Wundauflage soll der pflanzliche Extrakt Kamillenöl an der Oberfläche von 60 µm dicken Substraten 1 aus Silikon fixiert werden. Hierfür wurde zunächst auf einer Anzahl Substrate 1 eine 30 µm dicke Schicht 2 des Kamillenöls mittels Rakeln aufgetragen. Bei einer ersten Teilmenge an Substraten 1 (nachfolgend Referenzmenge genannt) wurde unmittelbar nach dem Auftragen der Schicht 2 das nicht am Substrat 1 haftende Kamillenöl mittels Ethanol abgewaschen. Mittels Transmissionsmessungen vor und nach dem Beschichten der Substrate mit dem Kamillenöl sowie nach dem Abwaschen des nicht am Substrat 1 haftenden Kamillenöls konnte ermittelt werden, dass bei der Referenzmenge lediglich durch das Auftragen des Kamillenöls im Mittel 13% der Schicht aus Kamillenöl am Substrat 1 haften bleibt.

Innerhalb einer Arbeitskammer 3 wurde bei einer zweiten Teilmenge Substrate 1 der Oberflächenbereich, auf dem die Schicht 2 aus Kamillenöl aufgetragen wurde, durch die Schicht 2 hindurch, mit beschleunigten, niederenergetischen Elektronen eines Elektronenstrahlgenerators 4 beaufschlagt. Dabei waren innerhalb der Arbeitskammer 3 Atmosphärenbedingungen eingestellt. Alternativ lässt sich das erfindungsgemäße Verfahren innerhalb einer Arbeitskammer auch bei Vakuumbedingungen oder bei Anwesenheit eines Gases, wie beispielsweise Stickstoff oder eines von der Atmosphäre abweichenden Gasgemisches, durchführen.

Der als Flächenstrahler ausgebildete Elektronenstrahlgenerator 4 wurde mit einer Beschleunigungsspannung von 150 kV bei einer Stromstärke von 2,5 mA betrieben und die Substrate 1 in verschiedenen Chargen mit einer Dosis, von 25 kGy, 50 kGy, 75 kGy bzw. 100 kGy bestrahlt. Alternativ kann das erfindungsgemäße Verfahren auch mit einem Linienstrahler oder Axialstrahler ausgeführt werden, deren Elektronenstrahlen über die Oberfläche eines Substrates hinweg abgelenkt werden.

Nach dem Beaufschlagen der Substrate 1 mit beschleunigten, niederenergetischen Elektronen wurde das nicht an den Substraten 1 haftende Kamillenöl ebenfalls mit Ethanol abgewaschen. Mittels Transmissionsmessungen konnte nachgewiesen werden, dass bei erfindungsgemäßer Vorgehensweise bei den angegebenen Chargen eine zunehmende Strahlendosis mit einer zunehmenden Haftung des Kamillenöls am Substrat einhergeht, wobei bei der Charge mit der Dosis von 100 kGy im Mittel 39% der Schicht aus Kamillenöl an den Substraten 1 haften bleibt.

Es sei angemerkt, dass die im Ausführungsbeispiel gemachten Angaben, wie Substratmaterial, Art des pflanzlichen Extraktes, Dicke des Substrates und der Schicht des pflanzlichen Extraktes sowie Parameter des Elektronenstrahlgenerators, lediglich beispielhaft sind und nicht das erfindungsgemäße Verfahren beschränken. Insbesondere eine geeignete Dosis für das Beaufschlagen einer Substratoberfläche mit beschleunigten Elektronen kann bei anderen Aufgabenstellungen andere Parameter erfordern. Welche Dosis bei einer anderen Aufgabenstellung insbesondere in Abhängigkeit vom Substratmaterial sowie der Art und Schichtdicke des pflanzlichen Extraktes am besten geeignet ist, lässt sich in Laborversuchen einfach ermitteln.

## Patentansprüche

1. Verfahren zum Fixieren pflanzlicher Wirkstoffe an einem Oberflächenbereich eines nichtmetallischen Substrates (1), **dadurch gekennzeichnet, dass** ein pflanzlicher Extrakt auf dem Oberflächenbereich des Substrates (1) aufgetragen und nachfolgend der Oberflächenbereich des Substrates (1) mit niederenergetischen Elektronen beaufschlagt wird.

2. Verfahren nach Anspruch 1, **dadurch gekennzeichnet, dass** beschleunigte Elektronen mit einer Energie von 100 keV bis 500 keV verwendet werden.

3. Verfahren nach Anspruch 1 oder 2, **dadurch gekennzeichnet, dass** das Beaufschlagen des Oberflächenbereiches des Substrates (1) mit niederenergetischen Elektronen mit einer Dosis von 1 kGy bis 200 kGy erfolgt.

4. Verfahren nach einem der vorhergehenden Ansprüche, **dadurch gekennzeichnet, dass** für das Substrat (1) ein Material verwendet wird, welches ein synthetisches Polymer, ein Biopolymer oder eine Textilie umfasst.

5. Verfahren nach einem der vorhergehenden Ansprüche, **dadurch gekennzeichnet, dass** der pflanzliche Extrakt mittels Drucken, Streichen, Rakeln, Sprühen oder mittels Eintauchen in den pflanzlichen Extrakt auf dem Oberflächenbereich des Substrates (1) aufgetragen wird.

## Claims

1. Method for fixing active plant ingredients on a surface region of a non-metallic substrate (1), **characterized in that** a plant extract is applied to the surface region of the substrate (1) and subsequently the surface region of the substrate (1) is treated with low-energy electrons.

2. Method according to Claim 1, **characterized in that** accelerated electrons having an energy of 100 keV to 500 keV are used.

3. Method according to Claim 1 or 2, **characterized in that** the surface region of the substrate (1) is treated with low-energy electrons in a dose of 1 kGy to 200 kGy.

4. Method according to any of the preceding claims, **characterized in that** the material used for the substrate (1) comprises a synthetic polymer, a biopolymer or a textile.

5. Method according to any of the preceding claims, **characterized in that** the plant extract is applied to the surface region of the substrate (1) by printing, spreading, knife coating or spraying or by immersion of the substrate into the plant extract.

## Revendications

1. Procédé pour la fixation de principes actifs végétaux au niveau d'une zone superficielle d'un substrat non métallique (1), **caractérisé en ce qu'**un extrait végétal est déposé sur la zone superficielle du substrat (1) et ultérieurement la zone superficielle du substrat (1) est soumise à des électrons de basse énergie.

2. Procédé selon la revendication 1, **caractérisé en ce que** des électrons accélérés dotés d'une énergie de 100 keV à 500 keV sont utilisés.

3. Procédé selon la revendication 1 ou 2, **caractérisé en ce que** la soumission de la zone superficielle du substrat (1) avec des électrons de basse énergie est réalisée avec une dose de 1 kGy à 200 kGy.

4. Procédé selon l'une quelconque des revendications précédentes, **caractérisé en ce que** pour le substrat (1), un matériau est utilisé qui comprend un polymère synthétique, un biopolymère ou un textile.

5. Procédé selon l'une quelconque des revendications précédentes, **caractérisé en ce que** l'extrait végétal est appliqué sur la zone superficielle du substrat (1) par pression, badigeonnage, raclage, pulvérisation ou par trempage dans l'extrait végétal.
